Europäisches Patentamt

European Patent Office

Office européen des brevets

(19)

(11) **EP 1 290 614 B1**

(12) **EUROPÄISCHE PATENTSCHRIFT**

(45) Veröffentlichungstag und Bekanntmachung des Hinweises auf die Patenterteilung:
**13.12.2006 Patentblatt 2006/50**

(21) Anmeldenummer: **01938122.7**

(22) Anmeldetag: **17.04.2001**

(51) Int Cl.:
**G06F 19/00** *(2006.01)*

(86) Internationale Anmeldenummer:
**PCT/EP2001/004335**

(87) Internationale Veröffentlichungsnummer:
**WO 2001/084459 (08.11.2001 Gazette 2001/45)**

(54) **VERFAHREN ZUR ERMITTLUNG SIGNIFIKANTER KNOCHENDICHTEVERLUSTE**

METHOD FOR DETERMINING SIGNIFICANT LOSSES IN BONE DENSITY

PROCEDE DE DETERMINATION DE PERTES DE DENSITE OSSEUSE IMPORTANTES

(84) Benannte Vertragsstaaten:
**AT BE CH CY DE DK ES FI FR GB GR IE IT LI LU MC NL PT SE TR**

(30) Priorität: **28.04.2000 DE 10020880**

(43) Veröffentlichungstag der Anmeldung:
**12.03.2003 Patentblatt 2003/11**

(73) Patentinhaber: **PES GESELLSCHAFT FÜR MEDIZINISCHE DIAGNOSESYSTEME MBH**
**04416 Leipzig-Markkleeberg (DE)**

(72) Erfinder: **BITTERLICH, Norman**
**09114 Chemnitz (DE)**

(74) Vertreter: **Seerig, Dieter**
**Patentanwälte**
**Findeisen Hübner Neumann Seerig**
**Pornitzstrasse 1**
**09112 Chemnitz (DE)**

(56) Entgegenhaltungen:
**WO-A-00/67031          WO-A-93/12255**
**WO-A-97/05553          DE-A- 10 020 880**

- **GARNERO P ET AL: "BIOCHEMICAL MARKERS OF BONE TURNOVER APPLICATIONS FOR OSTEOPOROSIS" ENDOCRINOLOGY AND METABOLISM CLINICS OF NORTH AMERICA, W.B. SAUNDERS COMPANY, PHILADELPHIA, US, Bd. 27, Nr. 2, 1988, Seiten 303-323, XP000938127 ISSN: 0889-8529 in der Anmeldung erwähnt**
- **RIIS B J ET AL: "BIOCHEMICAL MARKERS OF BONE TURNOVER TO MONITOR THE BONE RESPONSE TO POSTMENOPAUSAL HORMONE REPLACEMENT THERAPY" OSTEOPOROSIS INTERNATIONAL, XX, XX, Bd. 5, 1995, Seiten 276-280, XP000937862**
- **GARNERO P ET AL: "Les marqueurs biologiques du remodelage osseux: Variations pre-analytiques et recommandations pour leur utilisation." ANNALES DE BIOLOGIE CLINIQUE, Bd. 58, Nr. 6, November 2000 (2000-11), Seiten 683-704, XP008005560 ISSN: 0003-3898**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Ermittlung signifikanter Knochendichteverluste, wobei Messwerte von Bonemarkern aus Serum oder Urin ermittelt werden, die mit den Knochendichteverlusten assoziieren, zur mathematischen Verarbeitung zwecks Indikation der Knochendichteverluste verwendet werden.

Im Wechselspiel von Knochenab- und Knochenaufbauprozessen kann es zu überdurchschnittlichem Verlust von Knochensubstanz und -struktur kommen. Um diesen Verlust zu erfassen und signifikante Abweichungen von alters- und geschlechtsspezifischen Referenzwerten zu erkennen, werden im Allgemeinen Knochendichtemessungen (Osteodensitometrie) durchgeführt. Mit Methoden der Photonenabsortion bzw. der Computertomographie wird die Knochenmineraldichte an der Speiche (Radius), am Oberschenkelknochen (Femur) oder an der Lendenwirbelsäule (Vertebra lumbalis) gemessen. Unter der Annahme einer exponentiellen Entwicklung des Knochendichteschwundes kann aus Knochendichtewerten von mindestens drei Meßzeitpunkten die Verlustrate geschätzt werden. Die intraindividuellen Meßschwankungen betragen bis zu 5 %. Um diese Störeinflüsse auf die Ergebnisermittlung gering zu halten, sind die Abstände der Meßzeitpunkte so groß zu wählen, daß Veränderungen in der Knochendichte ausreichend nachweisbar sind. Aussagen liegen deshalb frühestens erst nach einem Jahr vor. Aus der WO 97/05553 A (HORUS THERAPEUTICS INC) 13. Februar 1997 ist ein Verfahren zur Ermittlung signifikanter Knochendichteverluste (Osteopenia) bekannt, wobei Messwerte für Bonemarker verwendet werden. Es wird besonders hervorgehoben, dass die Veränderungen von solchen Biomarkern dem Fachmann die Möglichkeiten eröffnen, die Veränderungen von Krankheiten zu erkennen. Die Verwendung von Neuronalen Netzen als mathematische Methode ermöglicht jedoch nur die Anwendung des vorgestellten Verfahrens bei statistisch verlgeichbaren Populationen in Bezug auf die Lerndatenmenge (Fig. 8). Von den vom Facharzt bekannten Einflüsse sind hierbei lediglich das Alter berücksichtigt. Das Verfahren ist zudem nur anwendbar, wenn das komplette Messprogramm unter vergleichbaren Messbedingungen ermittelt wird.

WO 93/12255 A (BARNHILL STEPHEN D) 24. Juni 1993 zeigt ein Verfahren auf, dass durch die Berechnung des Knochendichte-Koeffizienten ("bone density coefficient") charakterisiert ist. Diese Parameter sind methodenbedingt unmittelbar an die konkrete quanitative Bestimmung von Labormesswerte gebunden. Bekanntermaßsen ist die Variabilität von Labormessungen zwischen unterschiedlichen Laboreinrichtungen nicht zu vernachlässigen. Dies erfordert einen grundsätzlich anderen Lösungssatz, der insbesondere robust gegenüber Messschwankungen und flexibel in Bezug auf Adaption zum Messprogramm gestaltet ist.

Aufgabe der Erfindung ist es, ein Verfahren zur Ermittlung signifikanter Knochendichteverluste, wobei Messwerte von Bonemarkern aus Serum oder Urin ermittelt werden, die mit den Knochendichteverlusten assoziieren, zur mathematischen Verarbeitung zwecks Indikation der Knochendichteverluste verwendet werden, zu verbessern, um die Aussagekraft der Bonemarker zu erhöhen.

Erfindungsgemäß wird die Aufgabe dadurch gelöst, dass Messwerte von realen oder mathematisch simulierten Verlaufsprozessen von Knochendichteverlusten, die die zeitliche Abhängigkeit von Laborparametern zur praktisch oder theoretisch bekannten klinischen Symptomatik widerspiegeln, als Referenzwerte ermittelt werden und mit den Messwerten der Bonemarker in einen Datenspeicher geschrieben werden, und zum Analysezeitpunkt über eine Abfragefunktion alle verfügbaren patientenbezogenen Verlaufsdaten aus dem Datenspeicher kopiert und zur mathematischen Verarbeitung bereitgestellt werden, wobei

a) die Meßwerte der Bonemarker bezüglich der ersten Zeile in der Tabelle nach der Gleichung

$$M^*(t_n;k) = \frac{M(t_n;k) - M(t_l;k)}{M(t_l;k)}$$

k=1,...,K;n=1,...,N
normiert werden, und der zeitliche Verlauf der Messungen in Monate umgerechnet wird;

b) der normierte Meßwert in eine skalare Größe $D(t_n)$ zur graduierten Beschreibung des Knochendichteverlaufes umgewandelt wird, wobei als Funktion zur graduierten Beschreibung des Verlaufs die Beziehung

$$D(t_n) = \sqrt{\sum_{k=1}^{K} w_k \cdot \left( M^*(t_n;k) \right)^2}$$

verwendet wird;

c) aus den ermittelten Verlaufsbewertungen durch Interpolation Verlaufsbewertungen für diejenigen Zeitabschnitte nach

$$D^*(t) = \frac{(t_n - t) \cdot D(n-1) + (t - t_{n-1}) \cdot D(n)}{t_n - t_{n-1}} \quad , t \in [t_{n-1}, t_n]$$

berechnet werden, für die Referenzwerte verfügbar sind;

d) aus den interpolierten Verlaufsbewertungen Ähnlichkeitsmaßzahlen errechnet werden, wobei man zur Berechnung einer Ähnlichkeitsmaßzahl zwischen den zu untersuchenden Daten und allen auf dem Datenspeicher verfügbaren Referenzwerten die Funktion

$$A_j(t) = \sum_{m=1}^{M} \frac{t_m}{t_M} \cdot V_m \cdot \left( R_j(t_m - D^*(t_m)) \right)^2,$$

nutzt und dabei Ähnlichkeitsmaßzahlen zu den Referenzwerten und zu den Zeitpunkten in Monaten findet;

e) aus den Ähnlichkeitsmaßzahlen zu allen Referenzwerten solche Referenzwerte ermittelt werden, die im mathematischen Sinne eine hohe Ähnlichkeit aufweisen, wie die Ähnlichkeiten:

größte Ähnlichkeit

$$A^* = \min_{j=1,\dots J}\{A_j\}$$

positive Alternative (+)

$$A^+ = \min_{j=1,\dots,J, A_j \neq A^*, R_j(tN) > D(tN)}\{A_j\}$$

negative Alternative (-)

$$A^- = \min_{j=1,\dots,J, A_j \neq A^*, R_j(tN) < D(tN)}\{A_j\}$$

mit anschließender Ausgabe der Typ-Beschreibung als Textbaustein für die Situationsbeschreibung;

f) aus diesen drei Referenzverläufen die Vorhersage abgeleitet wird, wobei man den Vorhersagewert zum Zeitpunkt t die Größe

$$R(t) = \frac{1}{\sum_{i=1}^{3} B_i} \cdot \sum_{j=1}^{3}\left(\left(\sum_{i=1}^{3} B_i - B_j\right) \cdot R_j(t)\right)$$

verwendet, wenn $B_1 = A^*$, $B_2 = A^+$, $B_3 = A^-$ gesetzt sind;

g) die Freiheitsgrade bei der Spezifikation des Modells, gegeben als Funktionsparameter im funktionellen Zusammenhang von $D(t_n)$ und $A_j(t)$ durch Standardvorgaben belegt werden und durch statistische Analyse der Referenzwerte an die praktische Erfahrung zur Optimierung der quantitativen Vorhersage des Knochendichteverlustes angepaßt werden;

h) der Zeitpunkt errechnet wird, an dem nach dieser Vorhersagestrategie die prozentuale Abweichung größer als ein vorgegebener Schwellwert ist, wobei dieser Zeitpunkt Ausgangspunkt für die Planung des folgenden Untersuchungstermines ist.

**Weitere Ausführungsformen der Erfindung zeigen die Unteransprüche 2 bis 5.**

Vorteilhaft ist es, daß Freiheitsgrade, gegeben als Funktionsparameter im funktionellen Zusammenhang von $D(t_n)$ und $A_j(t)$; durch die mathematische Methode der kleinsten Fehlerquadrate so ausgefüllt werden, daß vorgegebene Reihenfolgen für Referenzwerte bestmöglichst berücksichtigt werden.

Die verwendeten Referenzwerte können Werte aus einem mathematisch-analytischen angenommenen Verlauf (Exponentialfunktion), erfahrungsbegründete Werte aus fiktiv angenommenen Verlaufsprozessen und konkrete Meßwerte von Patienten mit bekannten Verlaufssituationen sein.

Nachfolgend wird die Erfindung anhand eines Ausführungsbeispieles näher erläutert:

Als Bonemarker werden Osteocalcin, Parathormon und alkalische Phosphatase genutzt. Über verbreitete Labortechnik (HPLC, RIA, ELISA) werden aus Serum- oder Urinproben Meßwerte für die Bonemarker ermittelt. Dazu sind Schritte der Probenaufbereitung, wie

- Versetzen mit Antikörpern
- Inkubationsschritte
- Trennverfahren
- Einsetzen in Analysetechnik notwendig, um nach dem Meßvorgang einen quantitativen Wert in der parameterspezifischen Einheit als Monitor-/ Druckerausgabe oder als elektronisch verfügbarer Zahlenwert zu erhalten. Diese ermittelten Laborwerte werden über eine Eingabemaske auf einen elektronischen Datenspeicher geschrieben. Voraussetzung für das Verfahren ist, daß Referenzwerte bekannt sind. Referenzwerte können theoretisch berechnete Werte aus einem mathematisch-analytischen angenommenen Verlauf (Exponentialfunktion) oder erfahrungsbegründete Werte aus fiktiv angenommenen Verlaufsprozessen bzw. konkrete Meßwerte von Patienten mit bekannten Verlaufssituationen sein. Diese Referenzwerte liegen für gegebene Zeitpunkte vor und können nur im Rahmen des damit erfaßten Zeithorizontes in die Analyse eingehen. Im Ausführungsbeispiel wird die Exponentialfunktion $R(t) = a \cdot (1 - e^{-b \cdot t})$ für die Beschreibung der Knochendichteverluste benutzt. Die Parameter a und b beschreiben dann die Geschwindigkeit und den Grad des Verlustes (t in Monaten). Einige Referenzbeispiele sind in folgender Tabelle ausgeführt:

| Monate nach Erstmessung | Typ 0 a = 0 | Typ I a=1 b=0.01 | Typ II a=0.5 b=0.05 | Typ III a=0.5 b=0.1 | Typ IV a=2 b=0.01 | Typ V a=1 b = 0.05 | Typ VI a = 2 b = 0.05 |
|---|---|---|---|---|---|---|---|
| 0 | 0.00 | 0.00 | 0.00 | 0.00 | 0.00 | 0.00 | 0.00 |
| 6 | 0.00 | 0.06 | 0.13 | 0.23 | 0.12 | 0.26 | 0.52 |
| 12 | 0.00 | 0.11 | 0.23 | 0.35 | 0.23 | 0.45 | 0.90 |
| 18 | 0.00 | 0.16 | 0.30 | 0.42 | 0.33 | 0.59 | 1.19 |
| 24 | 0.00 | 0.21 | 0.35 | 0.45 | 0.43 | 0.70 | 1.40 |
| 30 | 0.00 | 0.26 | 0.39 | 0.48 | 0.52 | 0.78 | 1.55 |
| 36 | 0.00 | 0.30 | 0.42 | 0.49 | 0.60 | 0.83 | 1.67 |
| 42 | 0.00 | 0.34 | 0.44 | 0.49 | 0.69 | 0.88 | 1.76 |
| 48 | 0.00 | 0.38 | 0.45 | 0.50 | 0.76 | 0.91 | 1.82 |
| 54 | 0.00 | 0.42 | 0.47 | 0.50 | 0.83 | 0.93 | 1.87 |
| 60 | 0.00 | 0.45 | 0.48 | 0.50 | 0.90 | 0.95 | 1.90 |

Es folgen die Arbeitsschritte:

a) die ermittelten Meßwerte und von drei weiteren davorliegenden Meßzeitpunkten werden tabellarisch erfaßt;

| Lfd.Nr. | Datum | Osteocalcin in ug/l | PTH in ng/l | AP in U/L |
|---|---|---|---|---|
| 1 | 30.05.96 | 9.8 | 24.8 | 90 |
| 2 | 29.01.97 | 10.9 | 34.6 | 86 |
| 3 | 16.02.98 | 12.6 | 32.0 | 104 |
| 4 | 02.03.99 | 12.4 | 34.0 | 107 |

Meßwerte $M(t_n;k)$ für n = 1,..., 4 und k = 1,..., 3

b) die Meßwerte werden bezüglich der ersten Zeile in der Tabelle nach der Gleichung

$$M^*(t_n;k) = \frac{M(t_n;k) - M(t_1;k)}{M(t_1;k)}$$ normiert, und der zeitliche Abstand der Messungen wird in Monate umgerechnet.

| Lfd.Nr. | Monat | $M^*(t;1)$ | $M^*(t;2)$ | $M^*(t;3)$ |
|---------|-------|-----------|-----------|-----------|
| 1 | 0.0 | 0.00 | 0.00 | 0.00 |
| 2 | 8.0 | 0.11 | 0.40 | -0.04 |
| 3 | 20.6 | 0.29 | 0.29 | 0.16 |
| 4 | 33.1 | 0.27 | 0.37 | 0.19 |

Normierte Meßwerte $M^*(t_n;k)$ für n = 1,..., 4 und k = 1,..., 3

c) der normierte Meßwert wird in eine skalare Größe zur graduierten Beschreibung des Knochendichteverlustes umgewandelt, wobei als Funktion der graduierten Beschreibung des Verlaufes die Beziehung

$$D(t_n) = \sqrt{\sum_{k=1}^{K} w_k \cdot \left(M^*(t_n;k)\right)^2}$$

verwendet (K = 3; n = 1,...,3). Unter Standardansatz wird in natürlicher Weise die Festlegung w = 1 für alle Wichtungsfaktoren verstanden;

Verlaufsbewertungen zum Zeitpunkt t

d) Aus den ermittelten Verlaufsbewertungen werden durch Interpolation Verlaufsbewertungen für diejenigen Zeitabschnitte nach

$$D^*(t) = \frac{(t_n - t) \cdot D(n-1) + (t - t_{n-1}) \cdot D(n)}{t_n - t_{n-1}} \quad , t \in [t_{n-1}, t_n]$$

berechnet werden, für die Referenzwerte verfügbar sind;

Interpolierte Verlaufsbewertungen zu

fiktiven Meßzeitpunkten im 6-Monate-Abstand

e) aus den interpolierten Verlaufsbewertungen werden Ähnlichkeitsmaßzahlen errechnet, wobei man zur Berechnung einer Ähnlichkeitsmaßzahl zwischen den zu untersuchenden Daten und allen auf dem Datenspeicher verfügbaren Referenzwerten die Funktion

$$A_j(t) = \sum_{m=1}^{M} \frac{t_m}{t_M} \cdot V_m \cdot \left(R_j(t_m - D^*(t_m)\right)^2, j = 1,...,6; M = 6,$$

nutzt und dabei folgende Ähnlichkeitsmaßzahlen findet.

Unter Standardansatz wird in natürlicher Weise die Festlegung V=1 für alle Wichtungsfaktoren verstanden.

| Monat | | Typ 0 | Typ I | Typ II | Typ III | Typ IV | Typ V | Typ VI |
|-------|------|-------|-------|--------|---------|--------|-------|--------|
| 0 | | | | | | | | |
| 6 | 0.32 | 0.10 | 0.07 | 0.04 | 0.01 | 0.04 | **0.00** | 0.04 |
| 12 | 0.43 | 0.24 | 0.14 | 0.06 | 0.01 | 0.06 | **0.00** | 0.24 |
| 18 | 0.44 | 0.36 | 0.17 | 0.06 | **0.01** | 0.05 | 0.02 | 0.72 |
| 24 | 0.47 | 0.48 | 0.19 | 0.06 | **0.01** | 0.04 | 0.07 | 1.41 |

(fortgesetzt)

| Monat | | Typ 0 | Typ I | Typ II | Typ III | Typ IV | Typ V | Typ VI |
|---|---|---|---|---|---|---|---|---|
| 30 | 0.49 | 0.63 | 0.21 | 0.06 | **0.00** | 0.03 | 0.14 | 2.25 |

Ähnlichkeitsmaßzahlen zu allen Referenzwerten und zu allen Zeitpunkten bis 30 Monate.

Die Berechnungsvorschrift stellt ausdrücklich keine Schätzfunktion der Parameter a und b der Exponentialfunktion dar, da allgemeine Referenzwerte nicht dieser Vorschrift genügen müssen;

f) aus den Ähnlichkeitsmaßzahlen zu allen Referenzwerten solche Referenzwerte ermittelt werden, die im mathematischen Sinne eine hohe Ähnlichkeit aufweisen, wie die Ähnlichkeiten

größte Ähnlichkeit = 0,00

$$A^* = \min_{j=1,\ldots J}\{A_j\}$$

positive Alternative (+) = 0,03

$$A^+ = \min_{j=1,\ldots,J, A_j \neq A^*, R_j(t_N) > D(t_N)}\{A_j\}$$

negative Alternative (-) = 0,06

$$A^- = \min_{j=1,\ldots,J, A_j \neq A^*, R_j(t_N) < D(t_N)}\{A_j\}$$

Gemäß dieser Auswahlvorschrift wird der Typ III als der Referenzwert erkannt sowie der Typ IV und der Typ II als positiver bzw. negativer alternativer Referenzwert ausgewählt:

g) Aus diesen drei Referenzverläufen wird die Vorhersage abgeleitet. Setzt man zur Vereinfachung der Schreibweise $B_1 = A^*$, $B_2 = A^+$, $B_3 = A^-$, so wird als Vorhersagewert zum Zeitpunkt t die Größe

$$R(t) = \frac{1}{\sum_{i=1}^{3} B_i} \cdot \sum_{j=1}^{3}\left(\left(\sum_{i=1}^{3} B_i - B_j\right) \cdot R_j(t)\right)$$

verwandt.

h) die Freiheitsgrade bei der Spezifikation des Modelles, gegeben als Funktionsparameter im funktionellen Zusammenhang von $D(t_n)$ und $A_j(t)$; werden durch Referenzwerte ausgefüllt, um eine quantitative Vorhersage des Knochendichteverlustes zu erreichen;

| Monat | Vorhersage für Typ III | Vorhersage für Alternative (+) | Vorhersage für Alternative (-) | Vorhersage wert R (t) | Abweichung von Vorhersage in % |
|---|---|---|---|---|---|
| 36 | 0.49 | 0.60 | 0.42 | **0.52** | 6.1 |
| 42 | 0.49 | 0.69 | 0.44 | **0.55** | 12.2 |
| 48 | 0.50 | 0.76 | 0.45 | **0.58** | 16.0 |
| 54 | 0.50 | 0.83 | 0.47 | **0.61** | 22.0 |
| 60 | 0.50 | 0.90 | 0.48 | **0.63** | 26.0 |

Vorhersagewert

Die Aussagesicherheit dieser Vorhersage wird anhand der prozentualen Abweichung des mittleren Vorhersagewertes R(t) unter alleiniger Verwendung des mit den Ähnlichkeitsmaßwertzahlen $A_j$ ermittelten geeigneten Referenzwertes, im Ausführungsbeispiel Vorhersage für Typ III, der die größte Ähnlichkeit A* aufweist, charakterisiert.

i) Es wird der Zeitpunkt errechnet, an dem nach dieser Vorhersagestrategie die prozentuale Abweichung größer als ein vorgegebener Schwellwert ist, wobei dieser Zeitpunkt der Ausgangspunkt für die Planung des folgenden Untersuchungstermines ist. Dazu wird dieser Wert über Monitor oder Drucker ausgegeben sowie über Datenfernübertragung an den behandelnden Arzt übermittelt. Beim vorliegenden Ausführungsbeispiel wird der Schwellwert mit 10 % Abweichung angenommen. Man findet dann nach 40 Monaten nach der Erstmessung erstmalig eine größere Abweichung als 10 %. Der nächste Zeitpunkt für die Wiederholungsmessung sollte also nicht später als 7 Monate nach der Ermittlung der Meßwerte für die Bonemarker liegen.

| Monat | Vorhersage für Typ III | Vorhersage für Alternative (+) | Vorhersage für Alternative (-) | Vorhersage wert R (t) | Abweichung von Vorhersage i n % |
|---|---|---|---|---|---|
| 40 | 0.49 | 0.66 | 0.43 | 0.54 | 10.0 |

Abweichung der Vorhersagewerte größer als 10 %

**Patentansprüche**

1. Verfahren zur Ermittlung signifikanter Knochendichteverluste, wobei Messwerte von Bonemarkern aus Serum oder Urin ermittelt werden, die mit den Knochendichteverlusten assoziieren, zur mathematischen Verarbeitung zwecks Indikation der Knochendichteverluste verwendet werden, **dadurch gekennzeichnet, dass** Messwerte von realen oder mathematisch simulierten Verlaufsprozessen von Knochendichteveriusten, die die zeitliche Abhängigkeit von Laborparametern zur praktisch oder theoretisch bekannten klinischen Symptomatik widerspiegeln, als Referenzwerte ermittelt werden und mit den Messwerten der Bonemarker in einen Datenspeicher geschrieben werden, und zum Analysezeitpunkt über eine Abfragefunktion alle verfügbaren patientenbezogenen Verlaufsdaten aus dem Datenspeicher kopiert und zur mathematischen Verarbeitung bereitgestellt werden, wobei

a) die Meßwerte der Bonemarker bezüglich der ersten Zeile in der Tabelle nach der Gleichung

$$M^*(t_n;k) = \frac{M(t_n;k) - M(t_1;k)}{M(t_1;k)}$$

$k=1,...,K; n=1,...,N$
normiert werden, und der zeitliche Verlauf der Messungen in Monate umgerechnet wird;
b) der normierte Meßwert in eine skalare Größe $D(t_n)$ zur graduierten Beschreibung des Knochendichteverlaufes umgewandelt wird, wobei als Funktion zur graduierten Beschreibung des Verlaufs die Beziehung

$$D(t_n) = \sqrt{\sum_{k=1}^{K} w_k \cdot \left( M^*(t_n;k) \right)^2}$$

verwendet wird, die mittels des Wichtungsfaktors $W_R$ modifiziert wird
c) aus den ermittelten Verlaufsbewertungen durch Interpolation Verlaufsbewertungen für diejenigen Zeitabschnitte nach

$$D^*(t) = \frac{(t_n - t) \cdot D(n-1) + (t - t_{n-1}) \cdot D(n)}{t_n - t_{n-1}} \quad , t \in [t_{n-1}, t_n]$$

berechnet werden, für die Referenzwerte verfügbar sind;
d) aus den interpolierten Verlaufsbewertungen Ähnlichkeitsmaßzahlen errechnet werden, wobei man zur Berechnung einer Ähnlichkeitsmaßzahl zwischen den zu untersuchenden Daten und allen auf dem Datenspeicher

verfügbaren Referenzwerten $R_j$ (j=1,...J) die Funktion

$$A_j(t) = \sum_{m=1}^{M} \frac{t_m}{t_M} \cdot V_m \cdot \left( R_j(t_m) - D^*(t_m) \right)^2,$$

nutzt und dabei Ähnlichkeitsmaßzahlen zu den Referenzwerten und zu den Zeitpunkten in Monaten findet, wobei die Wertigkeit der Zeitabschnitte durch Verhältniszahlen $V_m$ (m = 1, ... M) gewichtet wird

e) aus den Ähnlichkeitsmaßzahlen zu allen Referenzwerten solche Referenzwerte ermittelt werden, die im mathematischen Sinne eine hohe Ähnlichkeit aufweisen, wie die Ähnlichkeiten:

größte Ähnlichkeit

$$A^* = \min_{j=1,...J} \{A_j\}$$

positive Alternative (+)

$$A^+ = \min_{j=1,...,J, A_j \neq A^*, R_j(t_N) > D(t_N)} \{A_j\}$$

negative Alternative (-)

$$A^- = \min_{j=1,...,J, A_j \neq A^*, R_j(t_N) < D(t_N)} \{A_j\}$$

mit anschließender Ausgabe der Typ-Beschreibung als Textbaustein für die Situationsbeschreibung;

f) aus diesen drei durch Schritt e) ausgewählten Referenzverläufen $R_j$ die Vorhersage abgeleitet wird, wobei man den Vorhersagewert zum Zeitpunkt t die Größe

$$R(t) = \frac{1}{\sum_{i=1}^{3} B_i} \cdot \sum_{j=1}^{3} \left( \left( \sum_{i=1}^{3} B_i - B_j \right) \cdot R_j(t) \right)$$

verwendet, wenn $B_1 = A^*$, $B_2 = A^+$, $B_3 = A^-$ gesetzt sind;

g) die Freiheitsgrade bei der Spezifikation des Modells, gegeben als Funktionsparameter im funktionellen Zusammenhang von $D(t_n)$ und $A_j(t)$ durch Standardvorgaben belegt werden und durch statistische Analyse der Referenzwerte an die praktische Erfahrung zur Optimierung der quantitativen Vorhersage des Knochendichteverlustes angepaßt werden;

h) der Zeitpunkt errechnet wird, an dem nach dieser Vorhersagestrategie die prozentuale Abweichung größer als ein vorgegebener Schwellwert ist, wobei dieser Zeitpunkt Ausgangspunkt für die Planung des folgenden Untersuchungstermines ist.

2. Verfahren nach Anspruch 1, **dadurch gekennzeichnet, daß** Freiheitsgrade, gegeben als Funktionsparameter im funktionellen Zusammenhang von $D(t_n)$ und $A_j(t)$; durch die mathematische Methode der kleinsten Fehlerquadrate so ausgefüllt werden, daß vorgegebene Reihenfolgen für Referenzwerte bestmöglichst berücksichtigt werden.

3. Verfahren nach Anspruch 1, **dadurch gekennzeichnet, daß** Referenzwerte verwendet werden, die berechnete Werte aus einem mathematisch-analytischen angenommenen Verlauf (Exponentialfunktionen) sind.

4. Verfahren nach Anspruch 1, **dadurch gekennzeichnet, daß** Referenzwerte verwendet werden, die erfahrungsbegründete Werte aus fiktiv angenommenen Verlaufsprozessen sind.

5. Verfahren nach Anspruch 1, **dadurch gekennzeichnet, daß** Referenzwerte verwendet werden, die konkrete Meßwerte von Patienten mit bekannten Verlaufssituationen sind.

**Claims**

1. Method for determining significant losses in bone density, in which measured values of bone markers from serum or urine are determined that are associated with the losses in bone density and are used for mathematical processing for the purpose of indicating the losses in bone density, **characterized in that** measured values of real or mathematically simulated bone density loss processes, which reflect the time function of laboratory parameters for practically or theoretically known clinical symptoms, are determined as reference values and are written with the measured values of the bone markers into a data memory and, at the time of analysis, all the available patient-related data are copied from the data memory by an interrogation function and made available for the mathematical processing, where

a) the measured values of the bone markers are normalized with respect to the first line in the table according to the equation

$$M^*(t_n;k) = \frac{M(t_n;k) - M(t_1;k)}{M(t_1;k)}$$

$k=1,...,K; n=1,...,N$
and the time profile of the measurements is converted into months;
b) the normalized measured value is converted into a scalar quantity $D(t_n)$ for the graduated description of the bone density profile, said graduated description of the profile being based on the equation

$$D(t_n) = \sqrt{\sum_{k=1}^{K} w_k \cdot \left(M^*(t_n;k)\right)^2}$$

which is modified by means of the weighting factor $W_k$, and
c) from the profile evaluations determined, the equation

$$D^*(t) = \frac{(t_n - t) \cdot D(n-1) + (t - t_{n-1}) \cdot D(n)}{t_n - t_{n-1}} \quad ,t \in [t_{n-1}, t_n]$$

is used to calculate, by interpolation, profile evaluations for those time sections for which reference values are available;
d) from the interpolated profile evaluations, similarity coefficients are calculated, the function

$$A_j(t) = \sum_{m=1}^{M} \frac{t_m}{t_M} \cdot V_m \cdot \left(R_j(t_m) - D^*(t_m)\right)^2,$$

being used to calculate a similarity coefficient between the data to be investigated and all the reference values available in the data memory, and similarity coefficients to the reference values and to the times in months are found, where the significance of the time sections is weighted by ratios $V_m$ (m=1, ... M),
e) from the similarity coefficients for all reference values, those reference values are determined which have a high similarity in the mathematical sense, such as the similarities:

greatest similarity

$$A^* = \min_{j=1,\ldots J}\{A_j\}$$

positive alternative (+)

$$A^+ = \min_{j=1,\ldots,J, Aj \neq A^*, Rj(tN) > D(tN)}\{A_j\}$$

negative alternative (-)

$$A^- = \min_{j=1,\ldots,J, Aj \neq A^*, Rj(tN) < D(tN)}\{A_j\}$$

with subsequent output of the type description as text component for describing the situation;

f) the prediction is derived from these three reference profiles Rj selected by step e), with the following expression

$$R(t) = \frac{1}{\sum\limits_{i=1}^{3} B_i} \cdot \sum\limits_{j=1}^{3}\left(\left(\sum\limits_{i=1}^{3} B_i - B_j\right) \cdot R_j(t)\right)$$

being used for the predicted value at time t if $B_1 = A^*$, $B_2 = A^+$, $B_3 = A$;

g) the degrees of freedom in the specification of the model, given as function parameters in the functional relation of $D(t_n)$ and $A_j(t)$, are verified by standard specifications and adapted, by statistical analysis of the reference values, to practical experience for optimizing the quantitative prediction of the bone density loss;

h) the time is calculated at which, according to this prediction strategy, the percentage deviation is greater than a predetermined threshold value, this time being the starting point for planning the next examination.

**2.** Method according to Claim 1, **characterized in that** degrees of freedom, given as function parameters in the functional relationship of $D(t_n)$ and $A_j(t)$, are filled by the mathematical method of the smallest error squares so that the best possible account is taken of predetermined sequences for reference values.

**3.** Method according to Claim 1, **characterized in that** reference values are used that are calculated values from a mathematically/analytically assumed profile (exponential functions).

**4.** Method according to Claim 1, **characterized in that** reference values are used that are empirical values from imaginary, assumed profiles.

**5.** Method according to Claim 1, **characterized in that** reference values are used that are concrete measured values from patients with known profile situations.

**Revendications**

**1.** Procédé pour déterminer des pertes significatives de densité osseuse, des valeurs mesurées de marqueurs osseux s'associant aux pertes de densité osseuse étant déterminées dans le sérum ou dans l'urine, et étant utilisées pour le traitement mathématique à des fins d'indication de pertes de la densité osseuse, **caractérisé en ce qu'**on détermine en tant que valeurs de référence des valeurs mesurées de processus de déroulement de pertes de la densité osseuse réels ou simulés mathématiquement, qui reflètent la dépendance dans le temps de paramètres de laboratoire par rapport à la symptomatique clinique connue dans la pratique ou dans la théorie et **en ce qu'**on les écrit dans une mémoire de données avec les valeurs mesurées des marqueurs osseux et **en ce qu'**au moment de l'analyse, on copie à partir de la mémoire de données toutes les données de déroulement disponibles relatives au patient via une fonction d'interrogation et on les met à disposition pour leur traitement mathématique, en

a) réduisant selon l'équation suivante

$$M^*(t_n; k) = \frac{M(t_n; k) - M(t_1; k)}{M(t_1; k)}$$

$k=1,...,K; n=1,...,N$

les valeurs mesurées des marqueurs osseux en ce qui concerne la première ligne dans le tableau et en convertissant la courbe de mesures dans le temps en mois ;

b) en transformant la valeur mesurée réduite en une grandeur scalaire $D(t_n)$ pour la description graduée de la courbe de la densité osseuse, en utilisant en tant que fonction pour la description graduée de la courbe la relation

$$D(t_n) = \sqrt{\sum_{k=1}^{K} w_k \cdot \left( M^*(t_n; k) \right)^2}$$

que l'on modifie au moyen du facteur de pondération $W_k$;

c) en calculant par interpolation selon

$$D^*(t) = \frac{(t_n - t) \cdot D(n-1) + (t - t_{n-1}) \cdot D(n)}{t_n - t_{n-1}} \quad , t \in [t_{n-1}, t_n]$$

à partir des évaluations de courbes déterminées des évaluations de courbes pour les périodes pour lesquelles des valeurs de référence sont disponibles;

d) en calculant à partir des évaluations de courbes interpolées, des indices de similitude en utilisant la fonction

$$A_j(t) = \sum_{m=1}^{M} \frac{t_m}{t_M} \cdot V_m \cdot \left( R_j(t_m) - D^*(t_m) \right)^2,$$

pour le calcul d'un indice de similitude entre les données à analyser et toutes les valeurs de référence disponibles dans la mémoire de données et en trouvant à cet effet des indices de similitude avec les valeurs de référence et les moments en mois, la valence des périodes étant pondérée par des nombres proportionnels Vm (m=1, ...M) ;

e) en recherchant à partir des indices de similitude avec toutes les valeurs de référence des valeurs de référence qui dans le sens mathématique font preuve d'une grande similitude, comme les similitudes :

plus grande similitude

$$A^* = \min_{j=1,...J} \{ A_j \}$$

alternative positive (+)

$$A^+ = \min_{j=1,...,J, A_j \neq A^*, R_j(tN) > D(tN)} \{ A_j \}$$

(suite)

alternative négative (-)

$$A^- = \min_{j=1,\ldots,J,\,A_j \neq A^*,\,R_j(tN) < D(tN)} \left\{ A_j \right\}$$

avec édition consécutive de la description de type en tant que module texte pour la description de la situation;

f) en dérivant la prévision grâce à l'étape e) à partir de ces trois courbes de référence $R_j$, en utilisant au moment t pour la valeur de prédiction la grandeur

$$R(t) = \frac{1}{\displaystyle\sum_{i=1}^{3} B_i} \cdot \sum_{j=1}^{3} \left( \left( \sum_{i=1}^{3} B_i - B_j \right) \cdot R_j(t) \right)$$

si $B_1 = A^*$, $B_2 = A^*$, $B_3 = A$ sont posés ;

g) en justifiant les degrés de liberté lors de la spécification du modèle, donnés en paramètre fonctionnel dans la connexité fonctionnelle de $D(I_n)$ et $A_j(t)$ par des prescriptions standard et en les adaptant à l'expérience pratique par analyse statistique des valeurs de référence, pour l'optimisation de la prédiction quantitative;

h) en calculant le moment auquel selon cette stratégie de prédiction, l'écart en pourcentage est supérieur à une valeur seuil prédéfinie, ce moment étant le point de départ pour la prévision de la prochaine date d'analyse.

2. Procédé selon la revendication 1, **caractérisé en ce qu'**on remplit par la méthode mathématique des plus petits carrés des erreurs des degrés de liberté, donnés en tant que paramètres fonctionnels dans la connexité fonctionnelle de $D(t_n)$ et $A_j(t)$, de façon à ce que des séquences prédéfinies pour des valeurs de référence soient prises en considération de la meilleure façon possible.

3. Procédé selon la revendication 1, **caractérisé en ce qu'**on utilise des valeurs de référence qui sont des valeurs calculées à partir d'une courbe supposée de façon mathématique analytique (fonctions exponentielles).

4. Procédé selon la revendication 1, **caractérisé en ce qu'**on utilise des valeurs de référence qui sont des valeurs à fondement expérimental à partir de processus de déroulement supposés de façon fictive.

5. Procédé selon la revendication 1, **caractérisé en ce qu'**on utilise des valeurs de référence qui sont des valeurs mesurées concrètes de patient avec des courbes connues.